(19) Europäisches Patentamt
European Patent Office
Office européen des brevets

(11) Veröffentlichungsnummer: **0 157 322 B1**

(12) # EUROPÄISCHE PATENTSCHRIFT

(45) Veröffentlichungstag der Patentschrift: **03.07.91**

(21) Anmeldenummer: **85103497.5**

(22) Anmeldetag: **25.03.85**

Die Akte enthält technische Angaben, die nach dem Eingang der Anmeldung eingereicht wurden und die nicht in dieser Patentschrift enthalten sind.

(51) Int. Cl.5: **G03C 1/34**, C07D 249/12, C07D 403/04, C07D 405/04, C07D 409/04, //(C07D403/04, 249:00,213:00),(C07D405/04, 307:00,249:00),(C07D409/04, 333:00,249:00)

(54) **Fotografisches Aufzeichnungsmaterial, Verfahren zur Stabilisierung fotografischer Aufzeichnungsmaterilien und neue Triazole.**

(30) Priorität: **06.04.84 DE 3412948**

(43) Veröffentlichungstag der Anmeldung: **09.10.85 Patentblatt 85/41**

(45) Bekanntmachung des Hinweises auf die Patenterteilung: **03.07.91 Patentblatt 91/27**

(84) Benannte Vertragsstaaten: **BE DE FR GB**

(56) Entgegenhaltungen:
**DE-A- 2 016 083**
**FR-A- 2 436 142**

**JOURNAL OF MEDICINAL CHEMISTRY, Band 8, Nr. 5, September 1965, Seiten 676-680, American Chemical Society, Washington, DC, US; D.H. JONES et al.: "Antiviral chemotherapy. I. The activity of pyridine and quinoline derivatives against neurovaccinia in mice"**

(73) Patentinhaber: **Agfa-Gevaert AG**

**W-5090 Leverkusen 1(DE)**

(72) Erfinder: **Öhlschläger, Hans, Dr.**
**Am Katterbach 34**
**W-5060 Bergisch Gladbach 2(DE)**
Erfinder: **Mäder, Helmut, Dr.**
**Theodor-Storm-Weg 1**
**W-5068 Odenthal(DE)**
Erfinder: **Sommer, Friedhelm, Dipl.-Chem.**
**Walter-Flex-Strasse 19**
**W-5090 Leverkusen 1(DE)**

**EP 0 157 322 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein fotografisches Aufzeichnungsmaterial mit einem Antischleiermittel (antifoggant), ein Verfahren zur Stabilisierung fotografischer Aufzeichnungsmaterialien sowie neue Triazole.

Aufzeichnungsmaterialien mit lichtempfindlichen Silberhalogenidemulsionen, insbesondere chemisch sensibilisierte, neigen bekanntlich zur Bildung von Schleiern, hervorgerufen durch Keime, die ohne Belichtung entwickelbar sind. Diese Schleierbildung tritt insbesondere auf bei zu langer Lagerung, besonders bei erhöhter Temperatur und Luftfeuchtigkeit.

Es ist bekannt, fotografischen Silberhalogenidemulsionen zur Verminderung der Schleierbildung sogenannte Antischleiermittel oder Stabilisierungsmittel zuzusetzen, z.B. heterocyclische Verbindungen, die Schwefel, beispielsweise in Form einer Mercaptogruppe, enthalten.

Beispielsweise sei hingewiesen auf die deutschen Auslegeschriften 1 183 371 (GB 1 067 066), DE 1 189 380 (US 3 364 028 und 3 365 294), DE 1 597 503 (US 3 615 617), DE 1 797 027, auf die deutschen Offenlegungsschriften 1 522 363 (GB 1 186 441), DE 2 042 533 (US 3 761 278), DE 2 130 031, DE 2 308 530 und DE 2 943 673 (US 4 264 721).

Aus dem US Patent 3 945 829 ist es bekannt, heterocyclische Mercaptoverbindungen einer kolloidales Silber enthaltenden Bindemittelschicht gegen Kontaktschleier zuzufügen.

Aus den DE-OS'en 1 906 952 und 1 908 217 (US 3 808 005 und 3 667 957) ist es bekannt, heterocyclische Verbindungen, die eine Carboxyl- oder Sulfogruppe aufweisen, zur Reduzierung des Schleiers in Aufzeichnungsmaterialien, z.B. in einer Silberhalogenidemulsionsschicht, zu verwenden.

Aus DE-OS 20 16 083 ist es bekannt, 1,2,4-Triazole mit substituierten Alkylmercaptogruppen in 3-Stellung, die in 5-Stellung eine Aminogruppe enthalten können, als Antischleiermittel einzusetzen.

Diese Verbindungen setzen aber in wirksamen Konzentrationen unter Umständen die Empfindlichkeit der stabilisierten Emulsion herab und können bei Einschleppung in das Bleich- oder Bleichfixierbad den Bleichvorgang hemmen, so daß ein Schleier durch nicht entferntes Silber entsteht.

Der Erfindung lag die Aufgabe zugrunde, fotografische Aufzeichnungsmaterialien mit mindestens einer Silberhalogenidemulsionsschicht gegen Schleier, insbesondere bei Lagerung bei höheren Temperaturen, zu stabilisieren, ohne die Bleichung zu beeinträchtigen.

Es wurde nun ein fotografisches Aufzeichnungsmaterial mit wenigstens einer lichtempfindlichen Silberhalogenidemulsionsschicht, gegebenenfalls weiteren Schichten und einem Antischleiermittel gefunden. Erfindungsgemäß ist im Material eine Verbindung folgender Formel I enthalten:

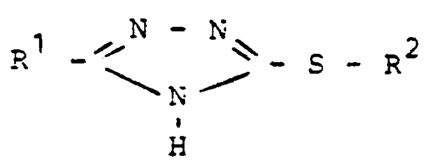

I

worin

$R^1$ Wasserstoff, einen Alkyl-, Cycloalkyl-, Alkoxyalkyl-, Alkylthio-, Aryl-, Amino-, substituierten Amino- oder einen heterocyclischen Rest,

$R^2$ einen unsubstituierten Alkylrest mit 5 bis 12 Kohlenstoffatomen bedeuten.

In einer bevorzugten Ausführungsform bedeutet

$R^1$ Wasserstoff, einen Alkylrest mit maximal 6-C-Atomen, einen Cycloalkylrest mit 5 oder 6 C-Atomen, einen Methoxymethylrest, einen Alkylthiorest mit maximal 6 C-Atomen, Phenyl, $NH_2$, Dimethylamino oder einen Heterocyclus mit 5 oder 6 Ringgliedern.

Geeignete heterocyclische Reste, für die $R^1$ stehen kann, sind z.B. Pyridyl, Furyl und Thienyl.

Weiterhin wurde ein Verfahren zur Stabilisierung fotografischer Aufzeichnungsmaterialien mit wenigstens einer Silberhalogenidemulsionsschicht und gegebenenfalls weiteren Schichten gefunden, bei dem man ein Antischleiermittel der Formel I so in das Material einbringt, daß es auf wenigstens eine Silberhalogenidemulsionsschicht wirken kann.

Ganz besonders bevorzugt sind die in Tabelle 1 angegebenen Verbindungen.

Tabelle 1

| Nr. | $R^1$ | $R^2$ |
|---|---|---|
| 1.1 | H | $C_6H_{13}$ |
| 1.2 | H | $C_8H_{17}$ |
| 1.3 | $NH_2$ | $(CH_2)_2-CH(CH_3)-CH_2-C(CH_3)_2-CH_3$ |
| 1.4 | $C_6H_5$ | $C_6H_{13}$ |
| 1.5 | 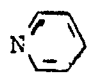 | $C_6H_{13}$ |
| 1.6 | 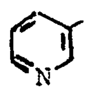 | $C_6H_{13}$ |
| 1.7 | $H_3C-O-CH_2-$ | $C_6H_{13}$ |
| 1.8 | 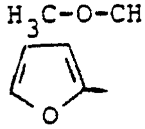 | $C_{10}H_{21}$ |
| 1.9 | 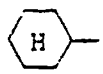 | $C_6H_{13}$ |
| 1.10 | $NH_2$ | $C_6H_{13}$ |
| 1.11 | 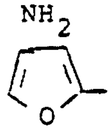 | $C_6H_{13}$ |
| 1.12 | $NH_2$ | $C_8H_{17}$ |
| 1.13 | 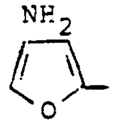 | $C_8H_{17}$ |
| 1.14 | 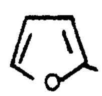 | $C_5H_{11}$ |
| 1.15 | 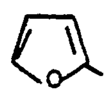 | $-CH_2-CH(C_2H_5)-C_4H_9$ |

Aus den deutschen Offenlegungsschriften 2 842 063, 2 937 127 und den US Patenten 4 259 437, 4 362 878 und 4 359 521 ist bekannt, DIR-Kuppler zu verwenden, die bei der Reaktion mit Entwickleroxidationsprodukten Mercaptotriazole zur Entwicklungsinhibierung frei setzen. Weil die Mercaptotriazole erst bei der Entwicklung entstehen, können sie im vorgelagerten Zeitraum, insbesondere bei der Lagerung, nicht wirksam werden. Nach der bildmäßigen Belichtung entstehen sie bei der Entwicklung nicht in gleichmäßiger

Verteilung sondern in Abhängigkeit von der Entwicklung.

Im Unterschied hierzu liegen die Mercaptotriazole gemäß der vorliegenden Erfindung im Aufzeichnungsmaterial von vorneherein vor, wirken also bei der Lagerung und sind gleichmäßig in der sie enthaltenden Schicht verteilt. Unter "Aufzeichnungsmaterialien" werden im Hinblick auf die vorliegende Erfindung Materialien verstanden, die zur Aufzeichnung bestimmt, aber noch nicht belichtet bzw. entwickelt sind.

Die erfindungsgemäßen Verbindungen können leicht durch Umsetzung der zugrundeliegenden 3-Mercapto-1,2,4-triazole mit Alkylhalogeniden unter Zusatz einer Base wie Triethylamin oder Ätznatron gewonnen werden.

Herstellung der Verbindung Nr. 1.1

28 g Ätzkali werden in 500 ml Methanol gelöst und die Lösung mit 50,5 g 3-Mercapto-1,2,4-triazol und 82,5 g Hexylbromid 12 Stunden unter Rückfluß erhitzt. Das ausgefallene Salz wird abfiltriert, die Lösung im Vakuum eingedampft. Der Rückstand wird aus Cyclohexan umkristallisiert. Man erhält 82,5 g 3-Hexylthio-1,2,4-triazol vom Schmelzpunkt 59-62 °C.

Analog können die übrigen Verbindungen hergestellt werden.

Die erreichbare Empfindlichkeit eines fotografischen Materials mit den erfindungsgemäßen Antischleiermitteln ist sehr hoch und wird während der Lagerung nicht vermindert. Die für den Fachmann sehr wichtige Stabilität eines fotografischen Materials wird bei der Anwendung der erfindungsgemäßen Verbindungen in hervorragender Weise erreicht. Der sonst bei der Entwicklung im allgemeinen auftretende Schleier wird unterdrückt.

Es ist günstig, die erfindungsgemäßen Verbindungen in Form von Lösungen zuzusetzen. Geeignet als Lösungsmittel sind beispielsweise niedere Alkohole, Tetrahydrofuran oder Aceton.

Die Emulsionen können in Kombination mit den erfindungsgemäßen weitere Antischleiermitteln und Stabilisatoren enthalten. Besonders geeignet sind Azaindene, vorzugsweise Tetra- oder Pentaazaindene, insbesondere solche, die mit Hydroxyl- oder Aminogruppen substituiert sind. Derartige Verbindungen sind z.B. in dem Artikel von Birr, Z.Wiss.Phot. 47,(1952), S. 2-58, beschrieben. Weitere geeignete Stabilisatoren und Antischleiermittel sind in der Zeitschrift Research Disclosure Nr. 17643 von Dezember 1978, Abschnitte I und II, veröffentlicht von Industrial Opportunities Ltd., Homewell Havant, Hampshire, P09 1 EF in Großbritannien angegeben.

Die Antischleiermittel können den lichtempfindlichen Silberhalogenidemulsionen vor der chemischen Reifung, zur chemischen Reifung oder nach der chemischen Reifung zugesetzt werden. In einer bevorzugten Ausführungsform werden sie nach der chemischen Reifung zur fertigen Gießlösung zugesetzt.

Die Stabilisierungsmittel werden vorzugsweise den lichtempfindlichen Silberhalogenidemulsionen vor oder nach der chemischen Reifung zugesetzt. Selbstverständlich kann man die Stabilisierungsmittel auch anderen fotografischen Schichten zusetzen die einer Silberhalogenidemulsionsschicht zugeordnet sind. Die Konzentration der Stabilisierungsmittel in der Emulsion kann innerhalb weiter Grenzen schwanken. Sie hängt von der Art der Emulsion und dem gewünschten Effekt ab. Im allgemeinen werden mit Mengen von 0,05 mmol bis 50 mmol, insbesondere von 0,2 bis 20 mmol pro Mol Silberhalogenid, die gewünschten Effekte erreicht.

Für die vorliegende Erfindung sind die üblichen Silberhalogenidemulsionen geeignet. Diese können als Silberhalogenid Silberchlorid, Silberbromid oder Gemische davon, eventuell mit einen geringen Gehalt an Silberjodid bis zu 10 Mol-%, enthalten.

Die Silberhalogenidemulsionen können mittels der üblichen Verfahrensweisen (z.B. Einfacheinlauf, Doppeleinlauf, mit konstantem oder beschleunigtem Stoffzufluß) hergestellt werden. Besonders bevorzugt ist die Herstellung nach dem Doppeleinlaufverfahren unter Steuerung des pAg-Wertes. Verwiesen wird auf die o.a. Research Disclosure Nr. 17643, Abschnitte I und II.

Die Silberhalogenidkörner können beispielsweise als Kuben, Oktaeder oder Tetradekaeder ausgebildet sein.

Die Emulsionen sind bevorzugt an der Kornoberfläche zu einer hohen Oberflächenempfindlichkeit chemisch sensibilisiert. Sie können nach bekannten Methoden chemisch sensibilisiert werden, z.B. mit aktiver Gelatine oder mit Verbindungen von Schwefel, Selen, Tellur, Gold, Palladium, Platin, Iridium, wobei die pAg-Werte zwischen 4 und 10, die pH-Werte zwischen 3, 5 und 9 und die Temperaturen zwischen 30 °C und 90 °C schwanken können; die chemische Sensibilisierung kann in Gegenwart von heterocyclischen Stickstoffverbindungen wie Imidazolen, Azaindenen, Azapyridazinen und Azapyrimidinen und Thiocyanatderivaten, Thioethern und anderen Silberhalogenidlösungsmitteln durchgeführt werden. Ersatzweise oder zusätzlich können die erfindungsgemäßen Emulsionen einer Reduktionssensibilisierung unterzogen werden, z.B. durch Wasserstoff, durch niedrigen pAg (z.B. kleiner als 5) und/oder hohen pH (z.B. über 8), durch Reduktionsmittel wie Zinn(II)chlorid, Thioharnstoffdioxid und Aminoborane.

Die Oberflächenreifkeime können auch als Troglodytenkeime (Suboberflächenkeime) gemäß der DE-OS

2 306 447 und der US-PS 3 966 476 vorliegen. Weitere Methoden sind beschrieben in der o.a. Research Disclosure Nr. 17643 im Abschnitt III.

Die Emulsionen können in an sich bekannter Weise optisch sensibilisiert werden. Verwiesen wird auf Ullmanns Encyclopädie der technischen Chemie, 4. Auflage, Band 18, Seiten 431 ff und auf die oben angegebene Research Disclosure Nr. 17643, Abschnitt IV. Die spektrale Sensibilisierung kann zu jedem Zeitpunkt der Emulsionsherstellung erfolgen, d.h. während oder nach der Silberhalogenidfällung und vor, während oder nach der chemischen Sensibilisierung.

Vorzugsweise handelt es sich bei dem erfindungsgemäßen Aufzeichnungsmaterial um ein farbfotografisches Aufzeichnungsmaterial. In einer bevorzugten Ausführungsform wird das Farbbild mit Hilfe von Farbkupplern erzeugt. Es ist möglich, den Farbkuppler erst bei der Entwicklung in das Aufzeichnungsmaterial eindiffundieren zu lassen.

In einer bevorzugten Ausführungsform enthält aber das fotografische Material selbst die üblichen Farbkuppler, die mit dem Oxidationsprodukt von Entwicklern, im allgemeinen p-Phenylendiaminen, unter Bildung von Farbstoffen reagieren können. Geeignete Kuppler sind beispielsweise bekannt aus den Veröffentlichungen "Farbkuppler" von W. Pelz in "Mitteilungen aus den Forschungslaboratorien der Agfa, Leverkusen/München", Band III, Seite 111 (1961), K. Venkataraman in "The Chemistry of Synthetic Dyes", Vol. 4, 341 bis 387, Academic Press (1971) und T.H. James, "The Theory of the Photographic Process", 4. Ed., S. 353-362, sowie aus der Research Disclosure Nr. 17643, Abschnitt VII.

Das Aufzeichnungsmaterial kann weiterhin DIR-Verbindungen enthalten. Unter DIR-Verbindungen werden derartige Verbindungen verstanden, die bei der Reaktion mit Farbentwickleroxidationsprodukten diffundierende organische Verbindungen in Freiheit setzen, die die Entwicklung von Silberhalogenid inhibieren. Die Inhibitoren können unmittelbar oder über nicht hemmende Zwischenverbindungen abgespalten werden. Verwiesen wird auf GB 953 454, US 3 632 345, US 4 248 962 und GB 2 072 363.

Die Farbkuppler und DIR-Verbindungen können in die erfindungsgemäßen Materialien nach üblichen, bekannten Methoden eingearbeitet werden.

Die erfindungsgemäßen Aufzeichnungsmaterialien enthalten vorzugsweise mindestens eine Silberhalogenidemulsionsschichten-Einheit für die Aufzeichnung von blauem, grünem und rotem Licht.

In einer bevorzugten Ausführungsform besteht wenigstens eine der Einheiten für die Aufzeichnung von grünem, rotem und blauem Licht aus wenigstens zwei Teilschichten. Es ist möglich, Teilschichten unterschiedlicher spektraler Sensibilisierung nach ihrer Empfindlichkeit zusammenzufassen.

Es können die üblichen Schichtträger verwendet werden. Verwiesen wird diesbezüglich auf die oben angegebene Research Disclosure Nr. 17643, Abschnitt XVII.

Als Schutzkolloid bzw. Bindemittel für die Schichten des Aufzeichnungsmaterials sind die üblichen hydrophilen filmbildenden Mittel geeignet, z.B. Proteine, insbesondere Gelatine, Alginsäure oder deren Derivate wie Ester, Amide oder Salze, Cellulose-Derivate wie Carboxymethylcellulose und Cellulosesulfate, Stärke oder deren Derivate oder hydrophile synthetische Bindemittel wie Polyvinylalkohol, teilweise verseiftes Polyvinylacetat, Polyvinylpyrrolidon und andere. Die Schichten können im Gemisch mit den hydrophilen Bindemitteln auch andere synthetische Bindemittel in gelöster oder dispergierter Form enthalten wie Homo- oder Copolymerisate von Acryl- oder Methacrylsäure oder deren Derivaten wie Estern, Amiden oder Nitrilen, ferner Vinylpolymerisate wie Vinylester oder Vinylether. Verwiesen wird weiterhin auf die in der oben angegebenen Research Disclosure 17643 in Abschnitt IX angegebenen Bindemittel.

Die Schichten des fotografischen Materials können in der üblichen Weise gehärtet sein, beispielsweise mit Härtern des Epoxidtyps, des heterocyclischen Ethylenimins und des Acryloyltyps. Weiterhin ist es auch möglich, die Schichten gemäß dem Verfahren der deutschen Offenlegungsschrift 2 218 009 zu härten, um farbfotografische Materialien zu erzielen, die für eine Hochtemperaturverarbeitung geeignet sind. Es ist ferner möglich, die fotografischen Schichten bzw. die farbfotografischen Mehrschichtenmaterialien mit Härtern der Diazin-, Triazin- oder 1,2-Dihydrochinolin-Reihe zu härten oder mit Härtern vom Vinylsulfon-Typ. Weitere geeignete Härtungsmittel sind aus den deutschen Offenlegungsschriften 2 439 551, 2 225 230, 2 317 672 und aus der oben angegebenen Research Disclosure 17643, Abschnitt XI bekannt.

In den erfindungsgemäßen fotografischen Materialien können außerdem noch weitere Substanzen enthalten sein, insbesondere Weichmacher, Netzmittel, Schirmfarbstoffe, Lichtstreumittel, Lichtreflexionsmittel, Gleitmittel, Antistatikmittel, Mattierungsmittel usw. Verwiesen wird auf die Research Disclosure 17643 und "Product Licensing Index" von Dezember 1971, Seiten 107 - 110.

Geeignete Farbentwicklersubstanzen für das erfindungsgemäße Material sind insbesondere solche vom p-Phenylendiamintyp, z.B. 4-Amino-N,N-diethyl-anilinhydrochlorid; 4-Amino-3-methyl-N-ethyl-N-β-(methansulfonamido)ethylanilinsulfathydrat; 4-Amino-3-methyl-N-ethyl-N-β-hydroxyethylanilinsulfat; 4-Amino-N-ethyl-N-(2-methoxyethyl)-m-toluidin-di-p-toluolsulfonsäure und N-Ethyl-N-β-hydroxyethyl-p-phenylendiamin. Weitere brauchbare Farbentwickler sind beispielsweise beschrieben in J.Amer.Chem.Soc. 73, 3100

(1951) und in G. Haist, Modern Photographic Processing, 1979, John Wiley and Sons, New York, Seiten 545 ff. Die Farbentwickler können die üblichen übrigen Bestandteile enthalten, z.B. Kalk- und Oxidationsschutzmittel und Mittel zur Schleierveränderung, wie z.B. Bromid oder an sich bekannte Stabilisatoren.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren, insbesondere z.B. Ethylendiamintetraessigsäure, Nitrilotriessigsäure, Iminodiessigsäure, N-Hydroxyethylethylendiamintriessigsäure, Alkyliminodicarbonsäuren und von entsprechenden Phosphonsäuren. Geeignet als Bleichmittel sind weiterhin Persulfate.

Beispiel 1

Eine hochempfindliche Silberbromidiodidemulsion mit 5 Mol-% Jodid und einem Gelatine-Silber-Verhältnis von 1:1,2 und einem Gehalt von 150 g Silber, berechnet als Silbernitrat pro kg Emulsion wurde mit Schwefel- und Goldverbindungen zur optimalen Empfindlichkeit gereift.

Der Emulsionsansatz wurde in mehrere Teile geteilt und pro kg Emulsion wurden folgende Substanzen zugesetzt:

| | |
|---|---|
| 4-Hydroxy-6-methyl-1,3,3a,7-tetraazainden<br>1 %ig, wäßrig-alkalische Lösung | 1500 mg |
| Saponin<br>10 %ig, gelöst in Wasser | 3,5 g |

und die aus der folgenden Tabelle ersichtlichen erfindungsgemäßen Substanzen (1 %ig gelöst in Methanol) in den angegebenen Mengen. Die Mengen wurden so bemessen, daß noch keine stärkere Beeinträchtigung der Empfindlichkeit auftrat.

Die Emulsionen wurden anschließend auf einem Celluloseacetatträger vergossen und getrocknet (Auftag 6,7 bis 7.0 g, berechnet als Silbernitrat pro $m^2$). Auf die Emulsionsschicht wurde jeweils eine Schutzschicht, die ein geeignetes Härtungsmittel und ein Netzmittel enthielt, mit einer Auftragsdicke von 2 g Gelatine/$m^2$ aufgetragen.

Die Proben wurden dann in einem Sensitometer hinter einem Graukeil belichtet und bei 38 °C 6 Minuten lang in einem Entwickler I der folgenden Zusammensetzung entwickelt. Dieser einen Silberkomplexbildner enthaltende Entwickler ist typisch für einen Schwarz-Weiß-Entwickler für die Umkehrverarbeitung von fotografischen Materialien.

Entwickler I

| | | |
|---|---|---|
| 4-Hydroxymethyl-4-methyl-1-phenyl-3-pyrazolidon | 1,4 | g |
| Natriumhydrogencarbonat sicc. | 12 | g |
| Diethylenglykol | 12 | ml |
| Kaliumhydroxid 45 % | 7 | ml |
| Kaliumjodid | 4,5 | mg |
| Hydrochinonsulfonsaures-Kalium | 22 | g |
| Kaliumcarbonat sicc. | 14 | g |
| Kaliumsulfit 45 % | 44 | ml |
| Kaliumrhodanid sicc. | 1 | g |
| Kaliumbromid | 2,2 | g |
| Nitrilo-trimethylentriphosphonsaures-$Na_6$-Salz | 1 | ml |

mit Wasser auf 1 l auffüllen und auf pH 9,6 einstellen.

Anschließend folgt ein Stoppbad bestehend aus 10 g Natriumacetat sicc. und 20 g 96 %igem Eisessig in 1 l Wasser. Anschließend wird mit 15 %iger Ammoniumthiosulfat- und 1 %iger Natriumsulfitlösung fixiert und danach gewässert. Die Ergebnisse der sensitometrischen Auswertung sind aus Tabelle 2 ersichtlich.

Zur Schleierprüfung wird je eine unbelichtete Probe 3 bzw. 6 Minuten lang in dem Entwickler I bei 38° C entwickelt.

Die weitere Verarbeitung erfolgt wie oben angegeben. Als Maß für die erhaltene Verschleierung wird die "prozentuale Verschleierung" in Tabelle 2 angegeben; die prozentuale Verschleierung ergibt sich aus dem Verhältnis von entwickeltem Silber (als Silbernitrat) dividiert durch Silber (als Silbernitrat) vor der Verarbeitung und Multiplikation dieses Verhältnisses mit 100.

Aus der Tabelle ist ersichtlich, daß die Substanzen den Schleier vermindern und daher als Antischleiermittel für Umkehrmaterialien geeignet sind.

Tabelle 2

| Verbindung Nr. | Menge Mol/Mol $AgNO_3$ | Empfindlichkeit | $\gamma$ | % Verschleierung nach 3 Min. | nach 6 Min. |
|---|---|---|---|---|---|
| Kontrollprobe | - | 42,0 | 0,86 | 26,5 | 55,6 |
| 1.8 | $3,5 \cdot 10^{-4}$ | 42,5 | 0,89 | 12,9 | 24,1 |
| 1.9 | $3,5 \cdot 10^{-4}$ | 42,4 | 0,84 | 21,0 | 44,2 |
| 1.9 | $7 \cdot 10^{-4}$ | 41,7 | 0,87 | 13,8 | 25,8 |
| 1.12 | $3,5 \cdot 10^{-4}$ | 42,2 | 0,83 | 14,9 | 35,7 |
| 1.12 | $7 \cdot 10^{-4}$ | 42,1 | 0,83 | 8,8 | 16,9 |
| 1.13 | $7 \cdot 10^{-4}$ | 42,1 | 0,86 | 9,6 | 25,3 |

Beispiel 2

Zu 1 kg einer grünsensibilisierten Bromjodsilber-Emulsion mit einem Silber (berechnet als Silbernitrat) / Gelatineverhältnis von 1:0,4 und einem Gehalt von 0,91 Mol Silberhalogenid pro kg Emulsion mit 5 Mol-% Jodid wurden 1,2 g 4-Hydroxy-6-methyl-1,3,3a,7-tetraazainden in wäßrig-alkalischer Lösung zugesetzt und die so erhaltene Emulsion in mehrere gleiche Teile geteilt und den einzelnen Proben die aus der folgenden Tabelle 3 ersichtlichen erfindungsgemäßen Verbindungen in Methanol gelöst in den angegebenen Mengen zugesetzt. Vor dem Beguß wurden der Emulsion pro kg zugesetzt:

75 g einer 5 %igen Gelatinelösung;

109 g einer 11,1 %igen Kupplerdispersion des folgenden Purpurkupplers

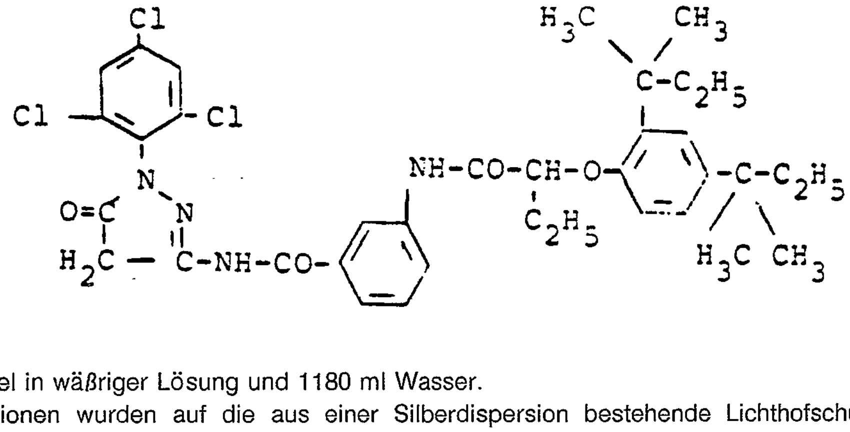

sowie Netzmittel in wäßriger Lösung und 1180 ml Wasser.

Die Emulsionen wurden auf die aus einer Silberdispersion bestehende Lichthofschutzschicht eines Celluloseacetatträgers mit einem Silberauftrag entsprechend 2,2 bis 2,3 g $AgNO_3/m^2$ vergossen.

Auf die Emulsionsschicht wurde jeweils eine Schutzschicht, die ein geeignetes Härtungsmittel und ein Netzmittel enthielt, mit einer Auftragsdicke von 2 g $Gelatine/m^2$ aufgetragen.

Die Proben wurden einer Frisch- und einer Heizschrankprüfung von 3 Tagen bei 60° C und 40 % rel. Luftfeuchtigkeit und einer Tropenschrankprüfung von 3 Tagen bei 35° C und 80 % rel. Luftfeuchtigkeit unterzogen.

Die Proben wurden dann in einem Sensitometer hinter einem Stufenkeil belichtet und in dem folgenden Entwickler II bei 38° C 3 1/4 Minuten entwickelt.

Entwickler II

| | |
|---|---|
| 1-Hydroxyethan-1,1-diphosphonsäure-$Na_2$-salz | 2 g |
| Ethylendiamin-N,N,N',N'-tetraessigsäure | 2 g |
| Kaliumcarbonat sicc. | 34,1 g |
| Natriumhydrogencarbonat sicc. | 1,55 g |
| Natriumdisulfit sicc. | 0,28 g |
| Natriumsulfit sicc. | 3,46 g |
| Kaliumbromid | 1,34 g |
| Hydroxylaminsulfat | 2,4 g |
| 4-Amino-3-methyl-N-ethyl-N-(ß-hydroxyethyl)-anilin | 4,7 g |

mit Wasser auf 1 l auffüllen.

Die weitere Verarbeitung umfaßt die folgenden Bäder:

| | |
|---|---|
| Stoppbad | 1 Minute bei 38°C; |
| Bleichbad | 3 1/4 Minuten bei 38°C; |
| Wässern | 3 1/2 Minuten bei 38°C; |
| Fixierbad | 3 1/4 Minuten bei 38°C; |
| Wässern | 5 Minuten bei 38°C. |

Die verwendeten Stopp-, Bleich- und Fixierbäder entsprechen den üblicherweise verwendeten (British Journal of Photography, 1974, Seiten 597 und 598).

Die erhaltenen Ergebnisse sind aus Tabelle 3 ersichtlich.

Die Substanzen vermindern den sehr hohen Schleier ohne die Empfindlichkeit und die Gradation wesentlich zu vermindern und verbessern die Lagerstabilität des fotografischen Materials.

Im Gegensatz dazu ergeben die aus DE-PS 1 906 952 bekannten Triazolderivate mit einer Sulfoethylthiogruppe (Vergleich I-IV) keine Schleierverminderung. Ebenfalls unwirksam sind Alkylthiotriazole mit zu kurzem Alkylrest (Vergleich V und VI).

Tabelle 3

| Verbindung Nr. | Mol/100g $AgNO_3$ | Frischeprüfung Empf. | Frischeprüfung Schleier | Tropenschrank Empf. | Tropenschrank Schleier |
|---|---|---|---|---|---|
| Kontroll-probe | - | 21,6 | 0,27 | 22,0 | 0,31 |
| 1.5 | $4 . 10^{-4}$ | 20,9 | 0,18 | 20,9 | 0,23 |
| 1.7 | $2 . 10^{-4}$ | 20,9 | 0,19 | 21,9 | 0,24 |
| | $4 . 10^{-4}$ | 20,5 | 0,15 | 21,5 | 0,20 |
| 1.8 | $4 . 10^{-4}$ | 20,5 | 0,15 | 21,0 | 0,19 |
| 1.9 | $4 . 10^{-4}$ | 20,3 | 0,14 | 21,0 | 0,17 |
| 1.10 | $4 . 10^{-4}$ | 21,2 | 0,15 | 21,8 | 0,24 |
| 1.11 | $4 . 10^{-4}$ | 20,6 | 0,17 | 21,5 | 0,22 |
| 1.12 | $2 . 10^{-4}$ | 21,3 | 0,21 | 21,9 | 0,24 |
| | $4 . 10^{-4}$ | 21,0 | 0,13 | 21,6 | 0,21 |
| 1.13 | $4 . 10^{-4}$ | 21,0 | 0,15 | 21,5 | 0,18 |
| Vergl. I | $2 . 10^{-4}$ | 21,4 | 0,24 | 22,1 | 0,29 |
| " II | $4 . 10^{-4}$ | 21,7 | 0,21 | 21,9 | 0,28 |
| " III | $4 . 10^{-4}$ | 21,9 | 0,28 | 22,2 | 0,30 |
| " IV | $2 . 10^{-4}$ | 21,3 | 0,23 | 21,8 | 0,28 |
| " V | $4 . 10^{-4}$ | 20,8 | 0,26 | 21,2 | 0,29 |
| " VI | $4 . 10^{-4}$ | 21.3 | 0,26 | 21,8 | 0,30 |

Vergleich I = Verbindung Nr. 22 aus DE-PS 1 906 952
Vergleich II = Verbindung Nr. 23 aus DE-PS 1 906 952
Vergleich III = Verbindung Nr. 26 aus DE-PS 1 906 952
Vergleich IV = Verbindung Nr. 30 aus DE-PS 1 906 952

Vergleich V = 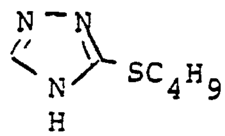

Vergleich VI = 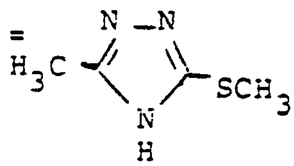

Beispiel 3

Zu 1 kg einer rotsensibilisierten Chlorbromsilberemulsion mit 15 Mol-% Chlorid und 100 g Silbernitrat pro kg wurden 64 mg 4-Hydroxy-6-methyl-1,3,3a,7-tetraazainden in wäßrigalkalischer Lösung zugesetzt und die so erhaltene Emulsion in mehrere Teile geteilt. Den einzelnen Proben wurden die aus der folgenden Tabelle 4 ersichtlichen erfindungsgemäßen Verbindungen, gelöst in Methanol, zugefügt. Anschließend wurden noch der folgende Blaugrünkuppler,

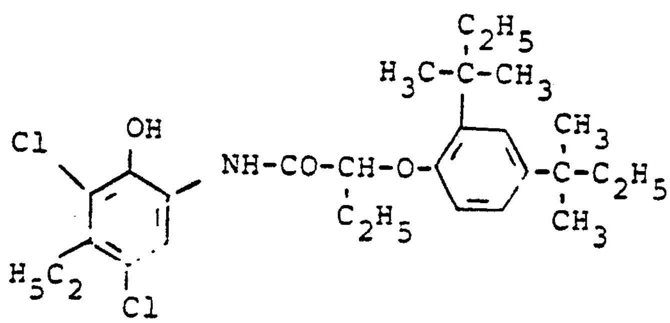

emulgiert in Gelatinelösung unter Verwendung von Tricresylphosphat, und ein Netzmittel zugesetzt.

Jeder dieser Ansätze wird auf ein substriertes polyethylenbeschichtetes Papier mit einem Auftrag von 0,48 g Silber berechnet als $AgNO_3/m^2$, aufgebracht. Auf die Emulsionsschicht wurde eine Schutzschicht mit 1,8 g $Gelatine/m^2$ aufgetragen.

Das Schichtpaket wurde dann mit einem geeigneten Härtungsmittel überschichtet.

Die so hergestellten Schichten wurden hinter einem Keil der Abstufung

$$\sqrt[3]{2}$$

und einem Rotfilter belichtet und dann in folgender Weise entwickelt:

| | | |
|---|---|---|
| Farbentwickler III | 33°C | 3,5 Minuten |
| Bleichfixierbad | 33°C | 1,5 Minuten |
| Wässerung | 33°C | 3 Minuten |

Die Verarbeitungsbäder wurden nach den folgenden Ansatzrezepten hergestellt:

Entwickler III

| | | |
|---|---|---|
| 900 | ml | Wasser |
| 15 | ml | Benzylalkohol |
| 15 | ml | Ethylenglykol |
| 3 | g | Hydroxylaminsulfat |
| 4,5 | g | 3-Methyl-4-amino-N-ethyl-N-(ß-methansulfonamidoethyl)-anilinsulfat |
| 32 | g | Kaliumcarbonat sicc. |
| 2 | g | Kaliumsulfit sicc. |
| 0,6 | g | Kaliumbromid |
| 1 | g | Dinatriumsalz der 1-Hydroxyethylidin-1,1-diphosphonsäure |

mit Wasser auf 1 l auffüllen und auf pH 10,2 einstellen.

Bleichfixierbad

| | | |
|---|---|---|
| 700 | ml | Wasser |
| 35 | ml | Ammoniaklösung (28 %ig) |
| 30 | g | Ethylendiamin-N,N,N',N'-tetraessigsäure |
| 15 | g | Natriumsulfit sicc. |
| 100 | g | Ammoniumthiosulfat sicc. |
| 60 | g | Natrium-(ethylendiamintetraacetat)-eisen-(III)-komplex |

mit Wasser auf 1 l auffüllen und auf pH 7 einstellen.

Nach der Entwicklung wurden die blaugrünen Farbkeile ausgemessen. Bei der Auswertung ergaben sich dann die Zahlenwerte der folgenden Tabelle 4.

Mit aufgeführt sind die Schleierwerte, die man erhält, wenn die Emulsion vor dem Beguß noch 24 Stunden lang bei 40 °C digeriert wird.

Tabelle 4

| Verbindung Nr. | konz. mg / 100 g $AgNO_3$ | $D_{Min.}$ Frische | $D_{Min.}$ 24 h Dig. | Empfindlichkeit D 0,6 | D 1,0 |
|---|---|---|---|---|---|
| Kontroll-probe | - | 0,167 | 0,202 | 1,63 | 1,46 |
| 1.1 | 30 | 0,145 | 0,150 | 1,67 | 1,50 |
| | 50 | 0,134 | 0,135 | 1,64 | 1,47 |
| 1.10 | 30 | 0,141 | 0,140 | 1,63 | 1,47 |
| | 60 | 0,134 | 0,135 | 1,60 | 1,40 |
| 1.11 | 50 | 0,145 | 0,155 | 1,59 | 1,43 |
| 1.12 | 50 | 0,136 | 0,146 | 1,56 | 1,37 |
| 1.13 | 50 | 0,146 | 0,155 | 1,57 | 1,41 |

Beispiel 4

Dieses Beispiel zeigt einen Vergleich bekannter mercaptogruppenhaltiger Stabilisatoren mit den erfindungsgemäßen bei der Bleichung.

Hierzu wird eine Silberfiltergelb-Gelatinelösung mit den in der Tabelle 5 angegebenen Mengen eines Stabilisators (Mol Stabilisator pro Mol Silber) versetzt, und nach Zufügen von Saponin als Netzmittel zu Schichten mit einer Dichte von 1.7-2.0 auf eine substrierte polyethylen beschichtete Papierunterlage vergossen.

Die Proben werden 5 Minuten lang in einer Pufferlösung bei pH 10 behandelt, 5 Minuten gewässert und dann in einem Bleichfixierbad der folgenden Zusammensetzung 1 Minute bei 20 °C gebadet:

20 g Na-Salz des Fe III-Komplexes der Ethylendiamintetraessigsäure
10 g Natriumsulfit sicc.
5 g Kaliumdihydrogenphosphat sicc.
100 g Ammoniumthiosulfat sicc.
mit Wasser auf 1 l auffüllen, pH 6,0.

Danach wurden die Keile eine Minute in fließendem Wasser gespült und getrocknet. Vor und nach der Behandlung wurde die Dichte der Keile gemessen, die Werte sind in der Tabelle 5 zusammengefaßt.

Tabelle 5

| Verbindung Nr. | Konzentration Mol/Mol Ag | Dichte unbehandelt | Dichte gebleicht |
|---|---|---|---|
| 1.1 | $6{,}9 \cdot 10^{-2}$ | 1,93 | 0,15 |
| 1.7 | $6{,}9 \cdot 10^{-2}$ | 1,72 | 0,13 |
| 1,8 | $7 \cdot 10^{-2}$ | 1,83 | 0,10 |
| 1.9 | $6{,}9 \cdot 10^{-2}$ | 1,73 | 0,11 |
| 1.10 | $7{,}1 \cdot 10^{-2}$ | 1,94 | 0,13 |
| 1.11 | $7{,}0 \cdot 10^{-2}$ | 1,87 | 0,11 |
| 1.12 | $7{,}0 \cdot 10^{-2}$ | 1,78 | 0,09 |
| 1.13 | $6{,}9 \cdot 10^{-2}$ | 1,80 | 0,09 |
| Vergleich A | $7{,}0 \cdot 10^{-2}$ | 1,93 | 2,06 |
| Vergleich B | $7{,}0 \cdot 10^{-2}$ | 1,97 | 1,96 |
| Vergleich C | $6{,}8 \cdot 10^{-2}$ | 1,97 | 2,06 |
| Kontrollprobe | - | 1,88 | 0,16 |

A = 1-Phenyl-5-mercaptotetrazol
B = 4-(p-Sulfoanilino)-5-methyl-3-mercapto-1,2,4-triazol (DE-PS 1 183 371)
C = 1-Phenyl-3-mercapto-1,2,4-triazol

## Ansprüche

1. Fotografisches Aufzeichnungsmaterial mit wenigstens einer lichtempfindlichen Silberhalogenidemulsionsschicht, gegebenenfalls weiteren Schichten und einem Antischleiermittel, dadurch gekennzeichnet, daß im Material eine Verbindung folgender Formel enthalten ist

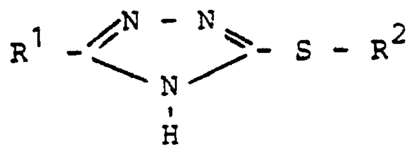

I

worin

$R^1$ Wasserstoff, einen Alkyl-, Cycloalkyl-, Alkoxyalkyl-, Alkylthio-, Aryl-, Amino-, substituierten Amino- oder einen heterocyclischen Rest,

$R^2$ einen unsubstituierten Alkylrest mit 5 bis 12 Kohlenstoffatomen bedeuten.

**2.** Fotografisches Aufzeichnungsmaterial nach Anspruch 1, dadurch gekennzeichnet, daß

$R^1$ Wasserstoff, einen Alkylrest mit maximal 6 C-Atomen, einen Cycloalkylrest mit 5 oder 6 C-Atomen, einen Methoxymethylrest, einen Alkylthiorest mit maximal 6 C-Atomen, Phenyl, $NH_2$, Dimethylamino oder einen Heterocyclus mit 5 oder 6 Ringgliedern bedeutet.

**3.** Fotografisches Aufzeichnugnsmaterial nach Anspruch 1, dadurch gekennzeichnet, daß

$R^1$ Pyridyl, Furyl oder Thienyl bedeutet.

**4.** Fotografisches Aufzeichnungsmaterial nach Anspruch 1, dadurch gekennzeichnet, daß wenigstens eine Verbindung der Formel I in einer Silberhalogenidemulsionsschicht enthalten ist.

**5.** Fotografisches Aufzeichnungsmaterial nach Anspruch 1, dadurch gekennzeichnet, daß wenigstens eine Verbindung der Formel I in einer eine Silberhalogenidemulsionsschicht zugeordneten Bindemittelschicht enthalten ist.

**6.** Fotografisches Aufzeichnungsmaterial nach Anspruch 1, dadurch gekennzeichnet, daß die Verbindung der Formel I in einer Menge von 0,05 mmol bis 50 mmol pro Mol der zugeordneten Silberhalogenidemulsionsschicht enthalten sind.

**7.** Verfahren zur Stabilisierung fotografischer Aufzeichnungsmaterialien mit wenigstens einer Silberhalogenidemulsionsschicht und gegebenenfalls weiteren Schichten, dadurch gekennzeichnet, daß man ein Antischleiermittel der in Anspruch 1 angegebenen Formel so in das Material einbringt, daß es auf wenigstens eine Silberhalogenidemulsionsschicht wirken kann.

## Claims

**1.** Photographic recording material having at least one light-sensitive silver halide emulsion layer, optionally other layers and an anti-foggant, characterised in that the material contains a compound corresponding to the following formula

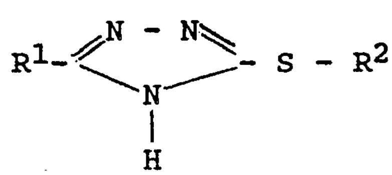

I

wherein

$R^1$ denotes hydrogen, an alkyl, cycloalkyl, alkoxyalkyl, alkylthio, aryl, amino or substituted amino or a heterocyclic group and

$R^2$ denotes an unsubstituted alkyl group having 5 to 12 carbon atoms.

**2.** Photographic recording material according to Claim 1, characterised in that

$R^1$ denotes hydrogen, an alkyl group having a maximum of 6 carbon atoms, a cycloalkyl group having 5 or 6 carbon atoms, a methoxymethyl group, an alkylthio group having a maximum of 6 carbon atoms, phenyl, $NH_2$, dimethylamino or a heterocyclic group having 5 or 6 ring

members.

3. Photographic recording material according to Claim 1, characterised in that $R^1$ denotes pyridyl, furyl or thienyl.

4. Photographic recording material according to Claim 1, characterised in that at least one compound corresponding to formula I is contained in a silver halide emulsion layer.

5. Photographic recording material according to Claim 1, characterised in that at least one compound corresponding to formula I is contained in a layer of binder associated with a silver halide emulsion layer.

6. Photographic recording material according to Claim 1, characterised in that the compound corresponding to formula I is present in a quantity of from 0.05 mmol to 50 mmol per mol of the associated silver halide emulsion layer.

7. Process for stabilizing photographic recording materials having at least one silver halide emulsion layer and optionally other layers, characterised in that an anti-foggant of the formula given in Claim 1 is introduced into the material in such a manner that it is capable of acting on at least one silver halide emulsion layer.

**Revendications**

1. Matériau d'enregistrement photographique avec au moins une couche d'émulsion d'halogénure d'argent photosensible, éventuellement d'autres couches et un agent anti-voile, caractérisé en ce que ce matériau contient un composé de la formule suivante:

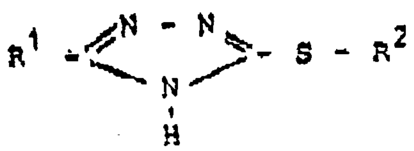

I

dans laquelle
- R1 représente un hydrogène, un radical alkyle, cycloalkyle, alcoxyalkyle, alkylthio, aryle, amino, amino substitué ou un radical hétérocyclique;
- R2 un radical alkyle non substitué avec 5 à 12 atomes de carbone.

2. Matériau photographique selon la revendication 1, caractérisé en ce que
- R1 représente de l'hydrogène, un radical alkyle avec maximum 6 atomes C, un radical cycloalkyle avec 5 ou 6 atomes de C, un radical méthoxyméthyle, un radical alkylthio avec maximum 6 atomes de C, un phényle, $NH_2$, un diméthylamino ou un hétérocycle avec 5 ou 6 éléments cycliques.

3. Matériau d'enregistrement Photographique selon la revendication 1, caractérisé en ce que R1 désigne un pyridyle, un furyle ou un thiényle.

4. Matériau d'enregistrement photographique selon la revendication 1, caractérisé en ce qu'au moins un des composés de la formule I set contenu dans la couche d'émulsion d'halogénure d'argent.

5. Matériau d'enregistrement photographique selon la revendication 1, caractérisé en ce qu'au moins un des composés de la formule I est contenu dans une couche de liant affectée à une couche d'émulsion d'halogénure d'argent.

6. Matérieu d'enregistrement photographique selon la revendication 1, caractérisé en ce que le composé de la formule I est contenu dans une quantité de 0,05 mmole à 50 mmoles par mole de la couche d'émulsion d'halogénure d'argent affectée.

7. Procédé de stabilisation des matériaux d'enregistrement photographiques avec au moins une couche d'émulsion d'halogénure d'argent et éventuellement d'autres couches, caractérisé en ce qu'on incorpore dans le matériau un agent anti-voile de la formule donnée à la revendication 1 dans le matériau de sorte qu'il puisse agir sur au moins une couche d'émulsion d'halogénure d'argent.